# EUROPEAN PATENT APPLICATION

(11) **EP 4 155 418 A1**
(43) Date of publication of application: **29.03.2023**
(21) Application number: 21198995.9
(22) Date of filing: 24.09.2021
(51) Int. Cl.: C12Q 1/6827, C12Q 1/6858

(54) **SINGLE NUCLEIC ACID FOR REAL-TIME DETECTION FOR SNP ANALYSIS OF APOE GENE AND DETECTION METHOD USING THE SAME**

(71) Applicant: Nuribio Co., Ltd., Sejong-si 30121 (KR)
(72) Inventor: NAM, Young Hyean, 16518 Gyeonggi-dodo (KR); KIM, Nam Hyo, 16892 Gyeonggi-do (KR); KIM, Hyun Mi, 16508 Suwon-si (KR)
(74) Representative: Mewburn Ellis LLP

(57) **Abstract**

The present disclosure relates to a single nucleic acid for real-time detection for SNP analysis of apolipoprotein E (ApoE) gene and a detection method using the same. More specifically, the present disclosure relates to a method of detecting in real time single nucleotide polymorphisms of ApoE gene by the use of a single nucleic acid, which has a structure of X-Y-Z (DNA-RNA-DNA) and consists of a nucleotide sequence capable of complementary binding to a portion or all of the nucleotide sequence of the ApoE gene showing single nucleotide polymorphisms, and a kit therefor.

The method for real-time detection of SNPs of the ApoE gene using a single nucleic acid that is cleaved only by a cleavage reagent according to the present disclosure has an advantage in that it may achieve a more accurate measurement compared to a conventional method of detecting genetic mutation using a probe. In addition, the method may rapidly and accurately distinguish the E2/E2, E3/E3, E4/E4, E2/E3, E2/E4 and E3/E4 phenotypes of the ApoE gene, and thus may be effectively used for diagnosis various diseases caused by ApoE genotypes, such as Alzheimer's disease and cardiovascular diseases, selection of therapeutic agents for these diseases, and prognostic diagnosis

## Description

### BACKGROUND

### 1. Technical Field

The present disclosure relates to a single nucleic acid for real-time detection for SNP analysis of apolipoprotein E (ApoE) gene and a detection method using the same. More specifically, the present disclosure relates to a method of detecting in real time single nucleotide polymorphisms of ApoE gene by the use of a single nucleic acid, which has a structure of X-Y-Z and consists of a nucleotide sequence capable of complementary binding to a portion or all of the nucleotide sequence of the ApoE gene showing single nucleotide polymorphisms at codons 112 and 158, and a kit therefor.

### 2. Related Art

Among genetic variations, variations caused by single nucleotide polymorphisms (SNPs) are the most common form and act as a cause of various diseases (Barreiro LB, et al., Methods Mol. Biol., 578:255-276, 2009; Beaudet L. et al., Genome Res., 11(4):600-608, 2001). Accordingly, a diagnostic method based on SNP detection for early diagnosis of various diseases caused by genetic variation is very efficient and enables rapid diagnosis. Therefore, many methods for accurately detecting SNPs have been proposed, and many studies thereon are still in progress (Ermini ML. et al., Biosen. & Bioele., 61:28-37, 2014; K. Chang et al., Biosen. & Bioele., 66:297-307, 2015).

Specifically, the most common methods that are used to analyze multiple genes include a method based on polymerase chain reaction (PCR), and a multiplex polymerase chain reaction (multiplex PCR) method.

The polymerase chain reaction has the advantage of being able to accurately amplify a target region of a gene to be detected by arbitrarily designing a primer or probe which is capable of binding to template DNA and to which a fluorophore and a quencher are bounded. However, the polymerase chain reaction can amplify and analyze only one gene of interest by a single reaction, and thus is inconvenient in that when multiple genes of interest need to be amplified, the same operation should be repeatedly performed.

The multiplex polymerase chain reaction has the advantage of being able to simultaneously analyze multiple gene regions by performing several polymerase chain reactions in a single tube. However, as several primers or probes are used simultaneously in a single tube, cross-reactions between primers or primers occur, and hence the number of gene regions that can be amplified at once is limited. In addition, the multiplex polymerase chain reaction has disadvantages in that it requires a lot of effort and time to find the reaction conditions and cannot provide good results in terms of sensitivity and specificity (Hardenbol P. et al., Nat. Biotechnol., 21(6):673-678, 2003).

In recent years, studies have been actively conducted to enable high throughput analysis by simultaneously amplifying multiple gene regions using universal primers without using multiplex polymerase chain reaction, and typical technologies include SNPlex capable of analyzing nucleotide polymorphisms (SNPs) of multiple gene regions, Goldengate assay, and molecular inversion probes (MIPs).

However, methods such as SNPlex, Goldengate assay, and molecular inversion probes (MIPs) have problems in that, since a portion of a product obtained in a first tube is transferred into and allowed to react in a second tube or several types of enzymes are used, contamination between different samples may occur, and the experimental method is complicated. In addition, these methods have a problem in that, since the same number of single nucleotide polymorphisms as the number of fluorescently labeled probes can be detected, the analysis cost increases as the number of single nucleotide polymorphisms to be analyzed increases.

Meanwhile, the apolipoprotein E (ApoE) gene is affiliated to apo C-I, C-II and LDL receptor genes, and ApoE gene polymorphisms have been found to be associated with the occurrence of various diseases such as hyperlipidemia and dementia.

The ApoE gene acts as a molecular mediator in the synthesis of β-amyloid protein in Alzheimer's patients, and ApoE4 is assumed as a strong mediator in this synthesis. In addition, it has also been reported that ApoE4 causes a change in the phosphorylation process due to a difference in binding form, thereby accelerating the formation of neurofibrillary tangles.

The human ApoE gene is located on the long arm of chromosome 19, and includes isoforms (E2, E3 and E4) of three alleles 2, 3 and 4, which are distinguished due to the difference in the 112^{th} and 158^{th} amino acids. Six different ApoE gene polymorphisms with different genotypes are formed by different combinations of these three alleles (see Table 7), and these polymorphisms are associated with the risk of cardiovascular disease and Alzheimer's disease.

Among them, ApoE4 protein is known to be an important risk factor for Alzheimer's disease and coronary artery disease, but ApoE2 protein has a preventive effect on Alzheimer's disease, whereas most patients with type III hypercholesterolemia are homozygous for ApoE2. The ApoE gene polymorphisms have important implications for cardiovascular disease and dementia, and thus detection thereof has been recognized as an important test not only for patients but also for healthy normal people.

Accordingly, the present inventors have long-term studied on a method capable of diagnosing diseases caused by ApoE gene variation simply and accurately by overcoming the low sensitivity and specificity in real-time detection of genetic variation caused by SNPs of the ApoE gene. As a result, the present inventors have found that when a single nucleic acid is used, it exhibits high sensitivity and high specificity, and thus is very useful in diagnosing various diseases caused by ApoE gene variation, such as Alzheimer's and cardiovascular diseases, thereby completing the present disclosure.

### [Prior Art Documents]

### [Non-Patent Documents]

Barreiro LB, et al., Methods Mol. Biol., 578:255-276, 2009
Beaudet L. et al., Genome Res., 11(4):600-608, 2001
Ermini ML. et al., Biosen. & Bioele., 61:28-37, 2014
K. Chang et al., Biosen. & Bioele., 66:297-307, 2015
Hardenbol P. et al. , Nat. Biotechnol., 21(6):673-678, 2003

### SUMMARY OF THE INVENTION

An object of the present disclosure is to provide a single nucleic acid for detecting single nucleotide polymorphisms of ApoE gene, the single nucleic acid being characterized in that: i) it has a structure of X-Y-Z; ii) it binds complementarily to a portion or all of the nucleotide sequence of the ApoE gene including single nucleotide polymorphism sites; and iii) at least two identical or different detectable markers are attached at both ends or inside of the single nucleic acid, wherein Y is an RNA consisting of one or two nucleotides located in the ApoE gene.

Another object of the present disclosure is to provide a single nucleic acid for detecting single nucleotide polymorphisms of ApoE gene, the single nucleic acid being characterized in that when it is used only as a probe for detecting single nucleotide polymorphisms (SNPs) of the ApoE gene, (a) X is a DNA consisting of 4 to 20 nucleotides, and (b) Z is a DNA consisting of 1 to 20 nucleotides.

Still another object of the present disclosure is to provide a single nucleic acid for detecting single nucleotide polymorphisms of ApoE gene, the single nucleic acid being characterized in that when it is used as both a primer and a probe, (c) X is a DNA consisting of 10 to 30 nucleotides, and (d) Z is a DNA consisting of 1 to 5 nucleotides.

Yet another object of the present disclosure is to provide a kit for real-time detection of single nucleotide polymorphisms of ApoE gene, the kit including the single nucleic acid.

Still another object of the present disclosure is to provide a method for detecting single nucleotide polymorphisms of ApoE gene, the method including steps of: a) isolating a target nucleic acid including ApoE gene single nucleotide polymorphism sites to be detected from a biological sample; b) producing type-1 single nucleic acid or type-2 single nucleic acid for detecting the ApoE gene single nucleotide polymorphisms; c) either mixing the target nucleic acid isolated in step a), the type-1 single nucleic acid produced in step b), an ApoE gene-specific primer set, and a cleavage reagent, or mixing the target nucleic acid isolated in step a), the type-2 single nucleic acid produced in step b), and a cleavage reagent, and then amplifying a target nucleic acid-single nucleic acid complex including the ApoE gene single nucleotide polymorphism sites by an extension reaction; and d) measuring the amount of a single nucleic acid fragment separated from the target nucleic acid-single nucleic acid complex including the ApoE gene single nucleotide polymorphism sites, amplified in step c).

To achieve the above objects, the present disclosure provides a single nucleic acid for detecting single nucleotide polymorphisms of ApoE gene.

Specifically, i) the single nucleic acid has a structure of X-Y-Z, ii) the single nucleic acid binds complementarily to a portion or all of the nucleotide sequence of ApoE gene including single nucleotide polymorphism sites, and iii) at least two identical or different detectable markers are attached at both ends or insides of the single nucleic acid, wherein Y is an RNA consisting of 1 or 2 nucleotides located in a single target gene and is cleaved by a cleavage reagent when hybridized with the ApoE gene.

In this case, when the single nucleic acid is used only as a probe for detecting single nucleotide polymorphisms (SNPs) of the ApoE gene, (a) X may be a DNA consisting of 4 to 20 nucleotides, (b) Z may be a DNA consisting of 1 to 20 nucleotides, and when the single nucleic acid is used as both a primer and a probe for detecting single nucleotide polymorphisms (SNPs) of the ApoE gene, (c) X may be a DNA consisting of 10 to 30 nucleotides, and (d) Z may be a DNA consisting of 1 to 5 nucleotides.

In addition, when the single nucleic acid is used only as a probe for detecting single nucleotide polymorphisms (SNPs) of the ApoE gene, Y may be cleaved by a cleavage reagent after hybridization with the ApoE gene, and X and Z may also be separated from the ApoE gene and may act as a probe. Alternatively, when the single nucleic acid is used as both a primer and a probe for detecting single nucleotide polymorphisms (SNPs) of the ApoE gene, Y may be cleaved by a cleavage reagent after hybridization with the ApoE gene, and Z may be separated from the ApoE gene, but X may act as both a primer and a probe without being separated.

The present disclosure also provides a kit for real-time detection of single nucleotide polymorphisms of ApoE gene.

The present disclosure also provides a method for detecting single nucleotide polymorphisms of ApoE gene, the method including steps of: a) isolating a target nucleic acid including ApoE gene single nucleotide polymorphism sites to be detected from a biological sample; b) producing type-1 single nucleic acid or type-2 single nucleic acid for detecting the ApoE gene single nucleotide polymorphisms; c) either mixing the target nucleic acid isolated in step a), the type-1 single nucleic acid produced in step b), an ApoE gene-specific primer set, and a cleavage reagent, or mixing the target nucleic acid isolated in step a), the type-2 single nucleic acid produced in step b), and a cleavage reagent, and then amplifying a target nucleic acid-single nucleic acid complex including the ApoE gene single nucleotide polymorphism sites by an extension reaction; and d) measuring the amount of a single nucleic acid fragment separated from the target nucleic acid-single nucleic acid complex including the ApoE gene single nucleotide polymorphism sites, amplified in step c).

### BRIEF DESCRIPTION OF THE DRAWINGS

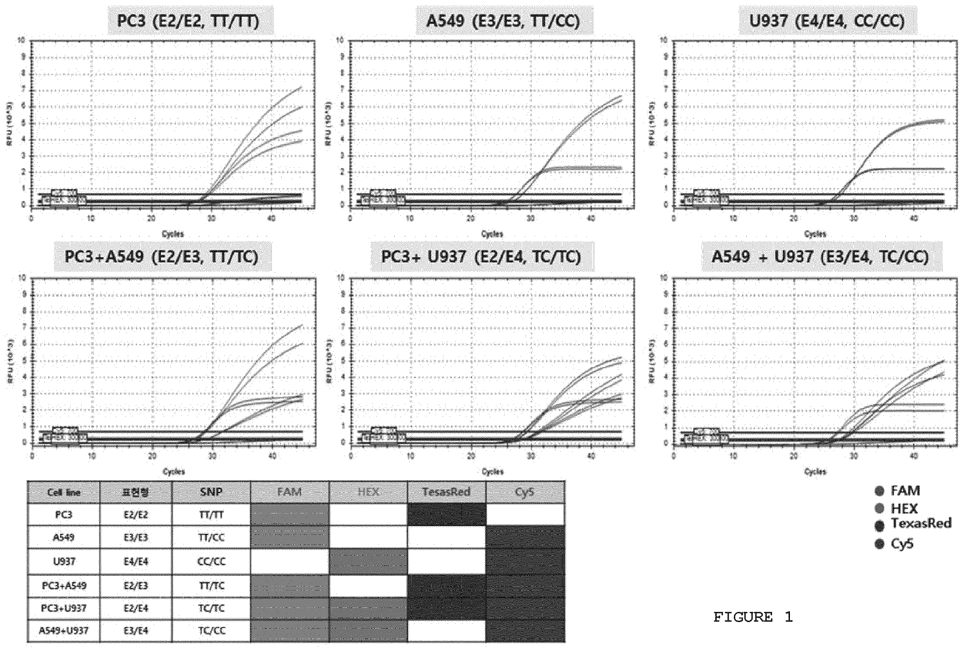

FIG. 1 shows the results of detecting ApoE gene single nucleotide polymorphisms using a single nucleic acid according to one example of the present disclosure, and specifically shows PCR results indicating that 6 different ApoE gene phenotypes (E2/E2, E3/E3, E4/E4, E2/E3, E2/E4, and E3/E4) may be distinguished using type-1 ApoE single nucleic acids (SEQ ID NOs: 3 to 6).

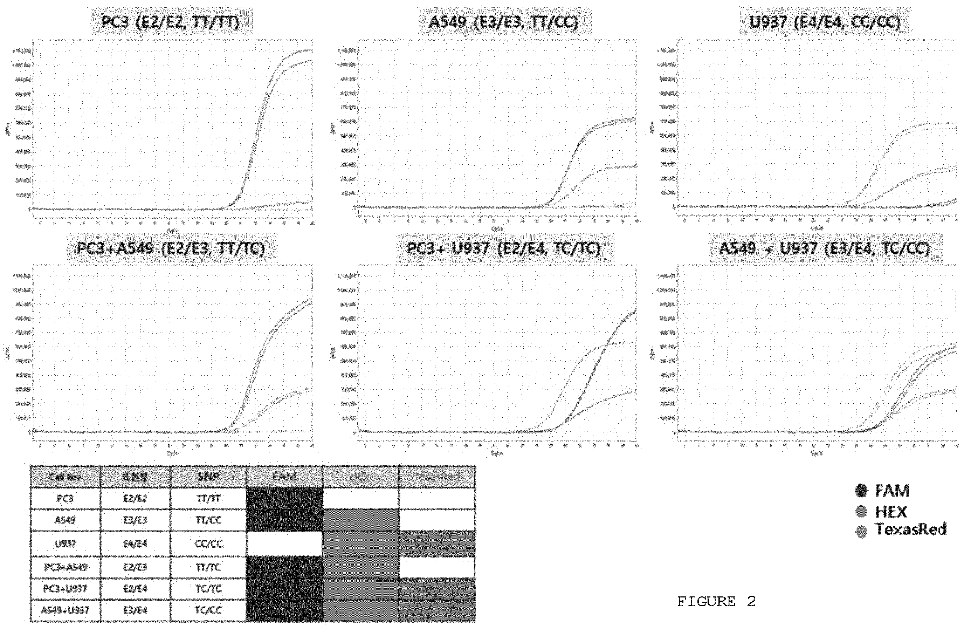

FIG. 2 shows the results of detecting ApoE gene single nucleotide polymorphisms using single nucleic acid according to one example of the present disclosure, and specifically shows PCR results indicating that 6 different ApoE gene phenotypes (E2/E2, E3/E3, E4/E4, E2/E3, E2/E4, and E3/E4) may be distinguished using type-2 ApoE single nucleic acids (SEQ ID NOs: 7 to 10).

### DETAILED DESCRIPTION

The present disclosure relates to a single nucleic acid for real-time detection for SNP analysis of apolipoprotein E (ApoE) gene and a detection method using the same. More specifically, the present disclosure relates to a method of detecting in real time single nucleotide polymorphisms of ApoE gene by the use of a single nucleic acid, which has a structure of X-Y-Z and consists of a nucleotide sequence capable of complementary binding to a portion or all of the nucleotide sequence of the ApoE gene showing single nucleotide polymorphisms, and a kit therefor.

In one aspect, the present disclosure provides a single nucleic acid for real-time detection of single nucleotide polymorphisms of ApoE gene.

In the present disclosure, the single nucleic acid is characterized in that: i) it has a structure of X-Y-Z; ii) it binds complementarily to a portion or all of the nucleotide sequence of ApoE gene including single nucleotide polymorphism sites; and iii) at least two identical or different detectable markers are attached at both ends or inside of the single nucleic acid.

In this case, the positions of the detectable markers attached are not limited to specific sites and may be any positions at which the detectable markers are separated when the region Y is cleaved by a cleavage reagent.

In addition, the single nucleic acid may form a complex by complementary binding to a portion or all of the nucleotide sequence of ApoE gene including the single nucleotide polymorphism sites to be detected in real time and amplify the complex.

As used herein, the term "single nucleic acid" refers to a nucleic acid for detecting single nucleotide polymorphisms (SNPs) of the ApoE gene. Here, a single nucleic acid that is used only as a probe in detection of single nucleotide polymorphisms (SNPs) may be referred to as "type-1 single nucleic acid" or "single nucleic acid type 1", and a single nucleic acid that is used as both a primer and a probe may be referred to as "type-2 single nucleic acid" or "single nucleic acid type 2". The type-1 single nucleic acid may act as a probe, and the type-2 single nucleic acid may be used as both a primer and a probe, unlike the type-1 single nucleic acid.

Specifically, when Y is cleaved by a cleavage reagent after the type-1 single nucleic acid hybridizes with the ApoE gene, X and Z may also be separated from the ApoE gene and may act as a probe, and when Y is cleaved after the type-2 single nucleic acid hybridizes with the ApoE gene, Z may be separated from the ApoE gene, but X may act as both a primer and a probe without being separated.

In one specific example, the type-1 single nucleic acid may consist of SEQ ID NOs: 3, 4, 5 and 6, and the type-2 single nucleic acid may consist of SEQ ID NO: 7, 8, 9 and 10.

The single nucleic acid of the present disclosure may have a structure of X-Y-Z, wherein each of X, Y and Z may have various numbers of nucleotides.

Y is an RNA consisting of 1 or 2 nucleotides located in the ApoE gene and is a region that is cleaved by a cleavage reagent.

Here, preferred examples of the cleavage reagent include enzymes such as DNase, RNase, helicase, exonuclease, and endonuclease, which mediate cleavage, but other known cleavage reagents may also be used.

X in the type-1 single nucleic acid is a DNA consisting of 4 to 20, preferably 4 to 19, more preferably 4 to 18, even more preferably 5 to 18, even more preferably 6 to 18, even more preferably 6 to 17, even more preferably 6 to 16, most preferably 6 to 15 nucleotides.

In one specific example, X in the type-1 single nucleic acid for detecting the SNP of codon 112 may be a nucleotide sequence consisting of 5'-GC GCG GAC ATG GAG GAC GTG-3' (SEQ ID NO: 27), and may also consist of a sequence including at least 4 nucleotides, preferably 4 to 20 nucleotides, counted from the 3' end of the nucleotide sequence of SEQ ID NO: 27. In a more specific example, the region X in the type-1 single nucleic acid for detecting the SNP of codon 112 may include any one of the sequences shown in Table 1 below.

**[Table 1]**

| SEQ ID NO | Sequence |
|---|---|
| 11 | 5'-C GTG-3' |
| 12 | 5'-AC GTG-3' |
| 13 | 5'-GAC GTG-3' |
| 14 | 5'-G GAC GTG-3' |
| 15 | 5'-AG GAC GTG-3' |
| 16 | 5'-GAG GAC GTG-3' |
| 17 | 5'-G GAG GAC GTG-3' |
| 18 | 5'-TG GAG GAC GTG-3' |
| 19 | 5'-ATG GAG GAC GTG-3' |
| 20 | 5'-C ATG GAG GAC GTG-3' |
| 21 | 5'-AC ATG GAG GAC GTG-3' |
| 22 | 5'-GAC ATG GAG GAC GTG-3' |
| 23 | 5'-G GAC ATG GAG GAC GTG-3' |
| 24 | 5'-CG GAC ATG GAG GAC GTG-3' |
| 25 | 5'-GCG GAC ATG GAG GAC GTG-3' |
| 26 | 5'-C GCG GAC ATG GAG GAC GTG-3' |
| 27 | 5'-GC GCG GAC ATG GAG GAC GTG-3' |

In another specific example, X in the type-1 single nucleic acid for detecting the SNP of codon 158 may be a nucleotide sequence consisting of 5'-AT GCC GAT GAC CTG CAG AAG-3' (SEQ ID NO: 54), and may also consist of a sequence including at least 4 nucleotides, preferably 4 to 20 nucleotides, counted from the 3' end of the nucleotide sequence of SEQ ID NO: 54, like X in the single nucleic acid for detecting the SNP of codon 112. In a more specific example, the region X in the type-1 single nucleic acid for detecting the SNP of codon 158 may include any one of the sequences shown in Table 1 below.

**[Table 2]**

| SEQ ID NO | Sequence |
|---|---|
| 38 | 5'-G AAG-3' |
| 39 | 5'-AG AAG-3' |
| 40 | 5'-CAG AAG-3' |
| 41 | 5'-G CAG AAG-3' |
| 42 | 5'-TG CAG AAG-3' |
| 43 | 5'-CTG CAG AAG-3' |
| 44 | 5'-C CTG CAG AAG-3' |
| 45 | 5'-AC CTG CAG AAG-3' |
| 46 | 5'-GAC CTG CAG AAG-3' |
| 47 | 5'-T GAC CTG CAG AAG-3' |
| 48 | 5'-AT GAC CTG CAG AAG-3' |
| 49 | 5'-GAT GAC CTG CAG AAG-3' |
| 50 | 5'-C GAT GAC CTG CAG AAG-3' |
| 51 | 5'-CC GAT GAC CTG CAG AAG-3' |
| 52 | 5'-GCC GAT GAC CTG CAG AAG-3' |
| 53 | 5'-T GCC GAT GAC CTG CAG AAG-3' |
| 54 | 5'-AT GCC GAT GAC CTG CAG AAG-3' |

In addition, X in the type-2 single nucleic acid is a DNA consisting of 10 to 30, preferably 11 to 30, more preferably 12 to 30, even more preferably 13 to 30, even more preferably 14 to 30, even more preferably 15 to 30, even more preferably 16 to 30, even more preferably 16 to 29, even more preferably 16 to 28, even more preferably 16 to 27, even more preferably 16 to 26, even more preferably 16 to 25, even more preferably 16 to 24, even more preferably 16 to 23, even more preferably 16 to 22, even more preferably 16 to 21, most preferably 16 to 20 nucleotides.

In one specific example, X in the type-2 single nucleic acid for detecting the SNP of codon 112 may be a nucleotide sequence consisting of 5'-GCC CGG CTG GGC GCG GAC ATG GAG GAC GTG-3' (SEQ ID NO: 37), and may also consist of at least 10 nucleotides, preferably 10 to 30 nucleotides, counted from the 3' end of the nucleotide sequence of SEQ ID NO: 37. In a more specific example, the region X in the type-2 single nucleic acid for detecting the SNP of codon 112 may include any one of the sequences shown in Table 3 below.

**[Table 3]**

| SEQ ID NO | Sequence |
|---|---|
| 17 | 5'-G GAG GAC GTG-3' |
| 18 | 5'-TG GAG GAC GTG-3' |
| 19 | 5'-ATG GAG GAC GTG-3' |
| 20 | 5'-C ATG GAG GAC GTG-3' |
| 21 | 5'-AC ATG GAG GAC GTG-3' |
| 22 | 5'-GAC ATG GAG GAC GTG-3' |
| 23 | 5'-G GAC ATG GAG GAC GTG-3' |
| 24 | 5'-CG GAC ATG GAG GAC GTG-3' |
| 25 | 5'-GCG GAC ATG GAG GAC GTG-3' |
| 26 | 5'-C GCG GAC ATG GAG GAC GTG-3' |
| 27 | 5'-GC GCG GAC ATG GAG GAC GTG-3' |
| 28 | 5'-GGC GCG GAC ATG GAG GAC GTG-3' |
| 29 | 5'-G GGC GCG GAC ATG GAG GAC GTG-3' |
| 30 | 5'-TG GGC GCG GAC ATG GAG GAC GTG-3' |
| 31 | 5'-CTG GGC GCG GAC ATG GAG GAC GTG-3' |
| 32 | 5'-G CTG GGC GCG GAC ATG GAG GAC GTG-3' |
| 33 | 5'-GG CTG GGC GCG GAC ATG GAG GAC GTG-3' |
| 34 | 5'-CGG CTG GGC GCG GAC ATG GAG GAC GTG-3' |
| 35 | 5'-C CGG CTG GGC GCG GAC ATG GAG GAC GTG-3' |
| 36 | 5'-CC CGG CTG GGC GCG GAC ATG GAG GAC GTG-3' |
| 37 | 5'-GCC CGG CTG GGC GCG GAC ATG GAG GAC GTG-3' |

In another specific example, X in the type-2 single nucleic acid for detecting the SNP of codon 158 may be a nucleotide sequence consisting of 5'-CTC CTC CGC GAT GCC GAT GAC CTG CAG AAG-3' (SEQ ID NO: 64), and may also consist of at least 4 nucleotides, preferably 4 to 20 nucleotides, counted from the 3' end of the sequence of SEQ ID NO: 64, like X in the type-2 single nucleic acid for detecting the SNP of codon 112. In a more specific example, the region X in the type-2 single nucleic acid for detecting the NMP of codon 158 may include any one of the sequences shown in Table 4 below.

**[Table 4]**

| SEQ ID NO | Sequence |
|---|---|
| 44 | 5'-C CTG CAG AAG-3' |
| 45 | 5'-AC CTG CAG AAG-3' |
| 46 | 5'-GAC CTG CAG AAG-3' |
| 47 | 5'-T GAC CTG CAG AAG-3' |
| 48 | 5'-AT GAC CTG CAG AAG-3' |
| 49 | 5'-GAT GAC CTG CAG AAG-3' |
| 50 | 5'-C GAT GAC CTG CAG AAG-3' |
| 51 | 5'-CC GAT GAC CTG CAG AAG-3' |
| 52 | 5'-GCC GAT GAC CTG CAG AAG-3' |
| 53 | 5'-T GCC GAT GAC CTG CAG AAG-3' |
| 54 | 5'-AT GCC GAT GAC CTG CAG AAG-3' |
| 55 | 5'-GAT GCC GAT GAC CTG CAG AAG-3' |
| 56 | 5'-C GAT GCC GAT GAC CTG CAG AAG-3' |
| 57 | 5'-GC GAT GCC GAT GAC CTG CAG AAG-3' |
| 58 | 5'-CGC GAT GCC GAT GAC CTG CAG AAG-3' |
| 59 | 5'-C CGC GAT GCC GAT GAC CTG CAG AAG-3' |
| 60 | 5'-TC CGC GAT GCC GAT GAC CTG CAG AAG-3' |
| 61 | 5'-CTC CGC GAT GCC GAT GAC CTG CAG AAG-3' |
| 62 | 5'-C CTC CGC GAT GCC GAT GAC CTG CAG AAG-3' |
| 63 | 5'-TC CTC CGC GAT GCC GAT GAC CTG CAG AAG-3' |
| 64 | 5'-CTC CTC CGC GAT GCC GAT GAC CTG CAG AAG-3' |

The region Z in the type-1 single nucleic acid is a DNA consisting of 1 to 20, preferably 2 to 20, more preferably 3 to 20, even more preferably 4 to 20, even more preferably 4 to 19, even more preferably 4 to 18, even more preferably 4 to 17, most preferably 4 to 16.

In one specific example, Z in the type-1 single nucleic acid for detecting the SNP of codon 112 may be a nucleotide sequence consisting of 5'-GCG GCC GCC TGG TGC AGT AC-3' (SEQ ID NO: 84), and may also consist of at least 4 nucleotides counted from the 5' end of the nucleotide sequence of SEQ ID NO: 84. In a more specific example, the region Z in the type-1 single nucleic acid for detecting the SNP of codon 112 may include any one of the sequences shown in Table 5 below.

**[Table 5]**

| SEQ ID NO | Sequence |
|---|---|
| 65 | 5'-G-3' |
| 66 | 5'-GC-3' |
| 67 | 5'-GCG-3' |
| 68 | 5'-GCG G-3' |
| 69 | 5'-GCG GC-3' |
| 70 | 5'-GCG GCC-3' |
| 71 | 5'-GCG GCC G-3' |
| 72 | 5'-GCG GCC GC-3' |
| 73 | 5'-GCG GCC GCC-3' |
| 74 | 5'-GCG GCC GCC T-3' |
| 75 | 5'-GCG GCC GCC TG-3' |
| 76 | 5'-GCG GCC GCC TGG-3' |
| 77 | 5'-GCG GCC GCC TGG T-3' |
| 78 | 5'-GCG GCC GCC TGG TG-3' |
| 79 | 5'-GCG GCC GCC TGG TGC-3' |
| 80 | 5'-GCG GCC GCC TGG TGC A-3' |
| 81 | 5'-GCG GCC GCC TGG TGC AG-3' |
| 82 | 5'-GCG GCC GCC TGG TGC AGT-3' |
| 83 | 5'-GCG GCC GCC TGG TGC AGT A-3' |
| 84 | 5'-GCG GCC GCC TGG TGC AGT AC-3' |

In another specific example, Z in the type-1 single nucleic acid for detecting the SNP of codon 158 may be a nucleotide sequence consisting of 5'-GCC TGG CAG TGT ACC AGG CC-3' (SEQ ID NO: 104), and may also consist of at least 4 nucleotides counted from the 5' end of the nucleotide sequence of SEQ ID NO: 104). In a more specific example, the region Z in the type-1 single nucleic acid for detecting the SNP of codon 158 may include any one of the sequences shown in Table 6 below.

**[Table 6]**

| SEQ ID NO | Sequence |
|---|---|
| 85 | 5'-G-3' |
| 86 | 5'-GC-3' |
| 87 | 5'-GCC-3' |
| 88 | 5'-GCC T-3' |
| 89 | 5'-GCC TG-3' |
| 90 | 5'-GCC TGG-3' |
| 91 | 5'-GCC TGG C-3' |
| 92 | 5'-GCC TGG CA-3' |
| 93 | 5'-GCC TGG CAG-3' |
| 94 | 5'-GCC TGG CAG T-3' |
| 95 | 5'-GCC TGG CAG TG-3' |
| 96 | 5'-GCC TGG CAG TGT-3' |
| 97 | 5'-GCC TGG CAG TGT A-3' |
| 98 | 5'-GCC TGG CAG TGT AC-3' |
| 99 | 5'-GCC TGG CAG TGT ACC-3' |
| 100 | 5'-GCC TGG CAG TGT ACC A-3' |
| 101 | 5'-GCC TGG CAG TGT ACC AG-3' |
| 102 | 5'-GCC TGG CAG TGT ACC AGG-3' |
| 103 | 5'-GCC TGG CAG TGT ACC AGG C-3' |
| 104 | 5'-GCC TGG CAG TGT ACC AGG CC-3' |

In addition, the region Z in the type-2 single nucleic acid is a DNA consisting of 1 to 5, preferably 1 to 4, more preferably 2 to 4 nucleotides.

In one specific example, Z in the type-2 single nucleic acid for detecting the SNP of codon 112 may be 5'-G-3' (SEQ ID NO: 65), 5'-GC-3' (SEQ ID NO: 66), 5'-GCG-3' (SEQ ID NO: 67), 5'-GCG G-3' (SEQ ID NO: 68), or 5-GCG GC-3' (SEQ ID NO: 69).

In another specific example, Z in the type-2 single nucleic acid for detecting the SNP of codon 158 may be 5'-G-3' (SEQ ID NO: 85), 5'-GC-3' (SEQ ID NO: 86), 5'-GCC-3' (SEQ ID NO: 87), 5'-GCC T-3' (SEQ ID NO: 88), or 5-GCC TG-3' (SEQ ID NO: 89) .

In the present disclosure, each of the single nucleotide polymorphisms (SNPs) may be detected specifically and sensitively according to the number of nucleotides constituting X and Z in each of the above-described single nucleic acids.

In one example of the present disclosure, it was confirmed through real-time PCR that 6 different ApoE gene phenotypes (E2/E2, E3/E3, E4/E4, E2/E3, E2/E4, and E3/E4) could be clearly distinguished using ApoE single nucleic acids (SEQ ID NOs: 3 to 6, and SEQ ID NOs: 7 to 10) (FIGS. 1 and 2).

In the present disclosure, each of the detectable markers may be either a fluorophore that binds to the single nucleic acid through a covalent or non-covalent bond, or a fluorescent pair composed of the fluorophore and a quencher.

The fluorophore may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670, and biotin, but is not necessarily limited thereto.

In addition, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2, and MGBNFQ, but is not necessarily limited thereto.

In the present disclosure, when the fluorescent pair is used as the detectable marker, the fluorophore and the quencher may be located in the region X or Z or the region Y, but is not limited thereto. In one example, the fluorophore may be located in X, and the quencher may be located in Y or Z.

In a process of detecting single nucleotide polymorphisms of the ApoE gene, the single nucleic acid of the present disclosure may be used as: i) a RT primer for synthesizing cDNA from RNA among nucleic acids; ii) a forward primer for amplifying a cDNA synthesized from nucleic acid (DNA or RNA); iii) a reverse primer for amplifying a cDNA synthesized from nucleic acid (DNA or RNA); iv) a forward primer and a reverse primer for amplifying a cDNA synthesized from nucleic acid (DNA or RNA); or v) a probe for real-time detection of a nucleic acid (DNA or RNA) to be detected.

In particular, the single nucleic acid of the present disclosure is used as a RT primer for synthesizing RNA (including miRNA, etc.) into cDNA, a forward primer for amplifying cDNA and a probe, or a reverse primer for amplifying cDNA and a probe, it is not necessary to construct the RT primer in a loop form during cDNA synthesis or form poly-A, and it is possible to synthesize cDNA by hybridization with an RNA to be detected and achieve amplification and real-time detection of a RNA (including miRNA, etc.) to be detected.

In another aspect, the present disclosure provides a kit for real-time detection of single nucleotide polymorphisms of ApoE gene, the kit including the single nucleic acid.

When the single nucleic acid of the present disclosure is used in a kit for detecting single nucleotide polymorphisms of ApoE gene, the kit may preferably further include an enzyme capable of cleaving the region Y of the single nucleic acid, in addition to the single nucleic acid.

In the present disclosure, as the enzyme capable of cleaving the region Y of the single nucleic acid, any enzyme may be used as long as it may specifically cleave the region Y of the single nucleic acid. For example, when the region Y is DNA, it is preferable to use DNA nuclease (DNase), particularly DNase I, DNase II, S1 nuclease, nuclease P1, AP endonuclease, or UvrABSC nuclease, and when the region Y is RNA, it is preferable to use ribonuclease (RNase), particularly RNase II, RNase III, RNase IV, RNaseH, or RNase T2.

When the single nucleic acid of the present disclosure is used in a kit for detecting single nucleotide polymorphisms of ApoE gene, the kit may further include reagents required for amplification of DNA, in addition to the single nucleic acid of the preset disclosure and an enzyme capable of cleaving the region Y of the single nucleic acid.

The reagents required for amplification include, for example, suitable amounts of DNA polymerase (e.g., thermostable DNA polymerase obtained from Thermusaquaticus (Taq), Thermusthermophilus (Tth), Thermus filiformis, Thermis flavus, Thermococcus literalis or Pyrococcus furiosus (Pfu)), DNA polymerase cofactor (Mg²⁺), buffer, dNTPs (dATP, dCTP, dGTP and dTTP) and water (dH₂O). In addition, the buffer includes suitable amounts of Triton X-100, dimethylsulfoxide (DMSO), Tween 20, nonidet P40, PEG 6000, formamide and bovine serum albumin (BSA), but is not limited thereto.

In another aspect, the present disclosure provides a method for detecting single nucleotide polymorphisms of ApoE gene, the method including steps of: a) isolating a target nucleic acid including ApoE gene single nucleotide polymorphism sites to be detected from a biological sample; b) producing type-1 single nucleic acid or type-2 single nucleic acid for detecting the ApoE gene single nucleotide polymorphisms; c) either mixing the target nucleic acid isolated in step a), the type-1 single nucleic acid produced in step b), an ApoE gene-specific primer set, and a cleavage reagent, or mixing the target nucleic acid isolated in step a), the type-2 single nucleic acid produced in step b), and a cleavage reagent, and then amplifying a target nucleic acid-single nucleic acid complex including the ApoE gene single nucleotide polymorphism sites by an extension reaction; and d) measuring the amount of a single nucleic acid fragment separated from the target nucleic acid-single nucleic acid complex including the ApoE gene single nucleotide polymorphism sites, amplified in step c).

Each of the method for real-time detection of single nucleotide polymorphisms of ApoE gene according to the present disclosure will be described in detail.

Step a) is a step of isolating a target nucleic acid including ApoE gene single nucleotide polymorphism sites to be detected from a biological sample.

In the present disclosure, the target nucleic acid including single nucleotide polymorphism site of ApoE gene may be RNA or DNA to be detected from a sample, or cDNA obtained by amplifying the RNA with a reverse transcription polymerase.

The sample may be either a biological sample or RNA, DNA, or fragment thereof isolated from the biological sample. Specifically, the sample may be any one or more selected from the group consisting of blood, saliva, urine, feces, tissue, cells and biopsy samples, or may be RNA, DNA, or fragment thereof isolated from a stored biological sample, but is not necessarily limited thereto.

The stored biological sample may be derived from a tissue stored for 1 week or more, 1 year or more, for example, 1 to 10 years, according to a conventional storage method known in the art, or a freeze-stored tissue, or a formalin-fixed tissue stored at room temperature.

In the present disclosure, extraction of RNA or DNA from the sample may be performed using various methods known in the art.

Step b) is a step of producing the single nucleic acid.

In the present disclosure, the single nucleic acid is as described above. Specifically, the single nucleic acid is characterized in that: i) it has a structure of X-Y-Z; ii) it binds complementarily to a portion or all of the nucleotide sequence of ApoE gene including single nucleotide polymorphism sites; and iii) at least two identical or different detectable markers are attached at both ends or inside thereof.

In the present disclosure, each of the detectable markers may be either a fluorophore that binds to the single nucleic acid by a covalent or non-covalent bond, or a fluorescent pair composed of the fluorophore and a quencher.

The fluorophore may be, for example, any one selected from the group consisting of Cy3, Cy5, Cy5.5, Bodipy, Alexa 488, Alexa 532, Alexa 546, Alexa 568, Alexa 594, Alexa 660, rhodamine, TAMRA, FAM, FITC, Fluor X, ROX, Texas Red, ORNAge green 488X, ORNAge green 514X, HEX, TET, JOE, Oyster 556, Oyster 645, Bodipy 630/650, Bodipy 650/665, Calfluor ORNAge 546, Calfluor red 610, Quasar 670, and biotin, but is not necessarily limited thereto. In addition, the quencher may be, for example, any one selected from the group consisting of DDQ-1, Dabcyl, Eclipase, 6-TAMRA, BHQ-1, BHQ-2, BHQ-3, Iowa Black RQ-Sp, QSY-7, QSY-2, and MGBNFQ, but is not necessarily limited thereto.

Step c) is a step of amplifying a target nucleic acid-single nucleic acid complex including single nucleotide polymorphism sites of the ApoE gene.

In the present disclosure, amplification of the target nucleic acid-single nucleic acid complex including single nucleotide polymorphism sites of the ApoE gene may be performed by an extension reaction after either mixing of the isolated target nucleic acid, the produced type-1 nucleic acid, the ApoE gene-specific primer set, and the cleavage reagent, or mixing of the target nucleic acid isolated in step a), the type-2 single nucleic acid produced in step b), and the cleavage reagent.

In the present disclosure, the ApoE gene-specific primer set may consist of SEQ ID NOs: 1 and 2, but is not particularly limited as long as it is a primer set having a nucleotide sequence complementary to the target nucleic acid including single nucleotide polymorphism sites of the ApoE gene.

In the present disclosure, the cleavage reagent is preferably an enzyme that mediates cleavage, but other known cleavage reagents may also be used. Here, in order to indicate RNA or DNA cleavage catalyzed by enzymes such as DNase, RNase, helicase, exonuclease and endonuclease, the term "enzyme-mediated cleavage" is used. In a preferred embodiment of the present disclosure, nicking and cleavage of the hybridized probe are more preferably performed by ribonuclease, which is an endonuclease or an exonuclease. The ribonuclease is more preferably a double-stranded ribonuclease that nicks and cleaves ribonucleic acid from a double-stranded DNA-RNA hybridization strand.

In the present disclosure, the cleavage reagent may be a ribonuclease (RNase) selected from among RNaseH, RNase II, RNase III, RNase IV and RNase T2.

Step d) is a step of measuring the amount of a single nucleic acid fragment separated from the target nucleic acid-single nucleic acid complex including the ApoE gene single nucleotide polymorphism sites, amplified in step c).

In the present disclosure, measurement of the amount of the single nucleic acid fragment may be performed using various detection methods. Specifically, the single nucleic acid fragment separated according to the present disclosure is preferably measured in real time or after completion of the extension reaction, and the measurement may be performed by measuring the change in fluorescence intensity or measuring chemiluminescence. The change in fluorescence intensity or chemiluminescence may be performed using any measurement device capable of detecting a fluorescent label known in the art, and for example, may be performed using a TRIAD Multimode Detector, a Wallac/Victor fluorescence or Perkin-Elmer LB50B luminescence spectrometer, a LightCycler96, Applied Biosystems 7500, or a Biorad CFX96 real-time PCR thermocycler, but is not limited thereto.

The method for measuring and detecting the amount of the single nucleic acid fragment cleaved according to the present disclosure may vary depending on the type of label or detectable marker introduced into the single nucleic acid or the reaction solution.

Since the identification of genetic mutation by the region Y of the single nucleic acid of the present disclosure becomes easy by the amplifying step following cleavage of the region Y, it is possible to identify the mutation through a subsequent nucleic acid amplification reaction. That is, when the region Y in the single nucleic acid having the genotypes to be identified according to the present disclosure has hybridized to the single nucleotide polymorphism sites of the target nucleic acid, the region Y is cleaved only when it has accurately bound complementarily to the single nucleotide polymorphism sites of the ApoE gene, and then an amplification reaction occurs. Hence, the single nucleotide polymorphisms of the ApoE gene may be clearly identified. Specifically, when the region Y in the single nucleic acid of the present disclosure does not bind complementarily to the single nucleotide polymorphism sites of the target nucleic even though the region Y has hybridized to the single nucleotide polymorphism sites, no amplification reaction occur because the site Y is not cleaved, and this means that the ApoE gene single nucleotide polymorphism sites to be measured in the target nucleic acid are not the genotypes to be identified.

The extension reaction that may be easily used in the present disclosure, that is, the nucleic acid amplification reaction, is known to those of ordinary skill in the art to which the present disclosure pertains. That is, a method for amplifying the target nucleic acid includes, but is not limited to, polymerase chain reaction (PCR), rolling circle amplification (RCA), strand displacement amplification (SDA), or nucleic acid sequence-based amplification (NASBA). The product of amplification of the nucleic acid is DNA or RNA.

In general, the reaction mixture contains the target nucleic acid, the single nucleic acid, components required for nucleic acid amplification reaction, and the cleavage enzyme so that amplification of the target nucleic acid and detection of single nucleotide polymorphisms by cleavage of the above-described single nucleic acid may be simultaneously performed. For each amplification reaction, it is necessary to individually optimize buffer conditions, primers, reaction temperature and single nucleic acid acid cleavage conditions. When the detection method of the present disclosure is used in conjunction with a nucleic acid amplification reaction, the sensitivity and speed of detecting the target nucleic acid will be remarkably improved.

Meanwhile, it was confirmed that the single nucleic acid for real-time detection of ApoE gene single nucleotide polymorphisms according to the present disclosure could rapidly and accurately detect ApoE gene single nucleotide polymorphisms associated with Alzheimer's disease and cardiovascular diseases. Thus, the single nucleic acid may be applied to a kit or a composition for diagnosing various diseases caused by ApoE genotypes, such as Alzheimer's disease and cardiovascular diseases, and may be effectively used to provide information on the occurrence of related diseases such as Alzheimer's disease by performing diagnosis of related diseases caused by ApoE genotypes in real time.

Hereinafter, the configuration and effects of the present disclosure will be described in more detail with reference to examples. However, these examples serve merely to illustrate the present disclosure, and the scope of the present disclosure is not limited to these examples.

### Example 1: ApoE Analysis Using Type-1 Single nucleic acids

Type-1 single nucleic acids were used to analyze 6 different phenotypes (E2/E2, E3/E3, E4/E4, E2/E3, E2/E4 and E3/E4) of Apolipoprotein E (ApoE) gene. The ApoE gene located on human chromosome 19 is a gene associated with cardiovascular diseases and Alzheimer's disease. The ApoE gene has three allele isoforms (*ApοE*ε2, *ApοE*ε3, and *ApοE*ε4) by DNA single nucleotide polymorphisms (SNPs) of codon 112 (Cys/Arg) and codon 158 (Cys/Arg) [genomic DNA positions 586 (T/C) and 724 (T/C)], and six different phenotypes (E2/E2, E3/E3, E4/E4, E2/E3, E2/E4, and E3/E4) are formed by different combinations of these alleles.

**[Table 7]**

| ApoE polymorphisms | Codon 112 | Codon 158 |
|---|---|---|
| ApoE2/2 | Cys (TGC) | Cys (TGC) |
| ApoE3/3 | Cys (TGC) | Arg (CGC) |
| ApoE4/4 | Arg (CGC) | Arg (CGC) |
| ApoE2/3 | Cys (TGC) | Cys (TGC)/Arg (CGC) |
| ApoE2/4 | Cys (TGC)/Arg (CGC) | Cys (TGC)/Arg (CGC) |
| ApoE3/4 | Cys (TGC)/Arg (CGC) | Arg (CGC) |

In order to distinguish six different phenotypes of the ApoE gene, the 5'-end was made possible to be analyzed by 4-plex assay using four different improved single nucleic acids (type-1 single nucleic acids) to each of which a fluorescent dye was attached. In a conventional analysis method, it was not easy to satisfy the sensitivity and specificity of the 4-plex assay, but the present disclosure showed satisfactory results therefor.

Specifically, in order to measure SNPs for the alleles of codon 112 and codon 158 of the ApoE gene, the type-1 single nucleic acids according to the present disclosure and primers were produced as shown in Table 8 below by IDT (Integrated DNA Technologies, USA). The type-1 single nucleic acid is a probe having a structure of X-Y-Z, and 6-FAM, HEX, TexasRed and Cy5 were attached to the 5'-ends of the single nucleic acids, respectively, and IABkFQ was attached to the 3'-end of each single nucleic acid. In addition, in order to distinguish ribonucleotide (RNA) from deoxyribonucleotide (DNA), the ribonucleotide (RNA) was marked by the subscript "r" in front of the sequence thereof. The type-1 single nucleic acids for detecting the SNP of codon 112 were SEQ ID NOs: 3 and 4, and the type 1 single nucleic acids for detecting the SNP of codon 158 were SEQ ID NOs: 5 and 6.

**[Table 8] ApoE-specific primer set and 4 different ApoE single nucleic acids (type 1)**

| Primer name/probe name | Sequence | SEQ ID NO |
|---|---|---|
| ApoE F primer | 5'-GAAGGCCTACAAATCGGAACT-3' | 1 |
| ApoE R primer | 5'-GCCACCTGCTCCTTCAC-3' | 2 |
| ApoE single nucleic acid 1 | 5-6-FAM/-GAGGACGTGrUGCGGCC-/3- IABkFQ | 3 |
| ApoE single nucleic acid 2 | 5-HEX/-GAGGACGTGrCGCGGCC-/3- IABkFQ | 4 |
| ApoE single nucleic acid 3 | 5-TexRd/-CTGCAGAAGrUGCCTGGCA-/3- IABkFQ | 5 |
| ApoE single nucleic acid 4 | 5-Cy5/-GCAGAAGrCGCCTGGCA-/3- IABkFQ | 6 |

For analysis, human cell lines PC3 (E2/E2), A549 (E3/E3) and U937 (E4/E4) were obtained from the Korea Cell Line Bank, and genomic DNA was isolated therefrom. For analysis of the six different phenotypes, as homozygous phenotypes, PC3 (E2/E2), A549 (E3/E3) and U937 (E4/E4) were used, and as heterologous phenotypes, PC3+A549 (E2/E3), PC3+U937 (E2/E4) and A549+U937 (E3/E4), which are hybrid types of genomic DNAs, were used at a high concentration of 32 ng (about 10⁴ copies) per reaction.

Polymerase chain reaction (PCR) was performed in the presence of 0.2 µM ApoE single nucleic acid 1, 0.15 µM single nucleic acid 2, 0.15 µM single nucleic acid 3, 0.075 µM single nucleic acid 4, 0.35 µM forward primer, 0.35 µM reverse primer, which are shown in Table 8 above, after the quantified genomic DNA, 4 µl of 0.5 U RNase-H, 4 µl of AptaTaq DNA Master (Roche) and 3 µl of GC-rich solution (Roche) were adjusted to a final volume of 20 µl with nuclease-free water. Here, the PCR was performed for 45 cycles, each consisting of 5 min at 95°C, 15 sec at 95°, and 70 sec at 65°C. The results are shown in FIG. 1.

As a result, it was confirmed that it was possible to analyze 6 different combinations of ApoE alleles in a single reaction well. From this result, it can be confirmed that the use of the improved probe type has an excellent ability to distinguish homozygous or heterologous single nucleotide polymorphisms.

### Example 2: ApoE Analysis Using Type-2 Single nucleic acid

In order to detect SNPs for the alleles of codon 112 and codon 158 of the ApoE gene, type-2 single nucleic acids according to the present disclosure were used. The type-2 single nucleic acids were produced as shown in Table 9 below by IDT (Integrated DNA Technologies, USA).

Here, each type-2 single nucleic acid has a structure of X-Y-Z and acts as a primer and a probe, and 6-FAM, HEX and TexasRed were attached to the 5'-ends of the single nucleic acids, respectively, and IABkFQ was attached to the 3'-end of each single nucleic acid. In addition, in order to distinguish ribonucleotide (RNA) from deoxyribonucleotide (DNA), the ribonucleotide (RNA) was marked by the subscript "r" in front of the sequence thereof. The type-2 single nucleic acids for detecting the SNP of codon 112 were SEQ ID NOs: 7 and 8, and the type-2 single nucleic acids for detecting the SNP of codon 158 were SEQ ID NOs: 9 and 10.

**[Table 9] Four different single nucleic acids (type 2)**

| Single nucleic acid name | Sequence | SEQ ID NO |
|---|---|---|
| ApoE single nucleic acid 1 | 5-6-FAM/-CGGTCATGGAGGACGTGrUGC- /3IABkFQ | 7 |
| ApoE single nucleic acid 2 | 5TexRd-XN/-GCGGATATGGAGGACGTrGCG-/3- IABkFQ | 8 |
| ApoE single nucleic acid 3 | 5-6-FAM/-CTGGTACACTGCCAGGCArCTT-/3- IABkFQ | 9 |
| ApoE single nucleic acid 4 | 5-HEX/-CTGGTACACTGCCAGGCrGATTC-/3- IABkFQ | 10 |

For analysis, human cell lines PC3 (E2/E2), A549 (E3/E3) and U937 (E4/E4) were obtained from the Korea Cell Line Bank, and genomic DNA was isolated therefrom. For analysis of the six different phenotypes, as homozygous phenotypes, PC3 (E2/E2), A549 (E3/E3) and U937 (E4/E4) were used, and as heterologous phenotypes, PC3+A549 (E2/E3), PC3+U937 (E2/E4) and A549+U937 (E3/E4), which are hybrid types of genomic DNAs, were used at a high concentration of 32 ng (about 10⁴ copies) per reaction.

Polymerase chain reaction (PCR) was performed in the presence of 0.375 µM ApoE single nucleic acid 1, 0.1 µM single nucleic acid 2, 0.25 µM single nucleic acid 3, and 0.25 µM single nucleic acid 4, which are shown in Table 9 above, after the genomic DNA, 0.1 ng of heat-resistant RNase, 4 µl of AptaTaq DNA Master w/o MgCl₂ (Roche), 2.75 mM MgCl₂, and 62.5 nM Low ROX were adjusted to a final volume of 20 µl with nuclease-free water. Here, the PCR was performed for 45 cycles, each consisting of 10 min at 95°C, 15 sec at 95°, and 55 sec at 64°C. The results are shown in FIG. 2.

As a result, it could be confirmed that, in the analysis of six combinations of ApoE alleles using the type-2 single nucleic acids according to the present disclosure, there were limitations that the 586T mutation of codon 112 and the 724T mutation of codon 158 were could be distinguished by the difference in end point fluorescence values by the same fluorescent dye, not by the difference in fluorescent dyes, but the type-2 single nucleic acids also had an excellent ability to distinguish homozygous or heterologous single nucleotide polymorphisms, like the type-1 single nucleic acids.

**As** described above, the method of detecting in real time single nucleotide polymorphisms of the ApoE gene by the use of the single nucleic acid according to the present disclosure has an advantage over the conventional SNP analysis method based on qPCR in that it may measure single nucleotide polymorphisms at two positions in a single space, and thus may achieve the measurement in a simpler and more accurate manner. That is, since the single nucleic acid according to the present disclosure is cleaved only by a cleavage reagent and may measure single nucleotide polymorphisms at two positions in a single space, it may achieve the measurement in a more accurate manner than a conventional method of ApoE gene SNPs using a probe.

In addition, when genetic mutations such as SNPs are analyzed using the single nucleic acid according to the present disclosure, it is possible to directly analyze the genetic mutations without the need for a separate confirmation process such as melting temperature analysis.

## Claims

1. A single nucleic acid for detecting single nucleotide polymorphisms of ApoE gene, the single nucleic acid being **characterized in that**:
i) it has a structure of X-Y-Z;
ii) it binds complementarily to a portion or all of the nucleotide sequence of the ApoE gene including single nucleotide polymorphism sites;
iii) at least two identical or different detectable markers are attached at both ends or inside of the single nucleic acid; and
iv) Y is an RNA consisting of 1 or 2 nucleotides located in the ApoE gene, hybridizes directly to the single nucleotide polymorphism sites in order to detect the single nucleotide polymorphisms (SNPs) of the ApoE gene, and is cleaved by a cleavage reagent when hybridized with the ApoE gene,
wherein, when the single nucleic acid is a type-1 single nucleic acid for detecting single nucleotide polymorphisms of the ApoE gene, X is a DNA consisting of 4 to 20 nucleotides, Z is a DNA consisting of 1 to 20 nucleotides, and X and Z are separated from the ApoE gene when Y is cleaved by the cleavage reagent after hybridization between the ApoE gene and the single nucleic acid; and
when the single nucleic acid is a type-2 single nucleic acid for detecting single nucleotide polymorphisms of the ApoE gene, X is a DNA consisting of 10 to 30 nucleotides, Z is a DNA consisting of 1 to 5 nucleotides, and Z is separated from the ApoE gene when Y is cleaved by the cleavage reagent after hybridization between the ApoE gene and the single nucleic acid, but X acts as a primer and a probe without being separated.

2. The single nucleic acid of claim 1, which consists of SEQ ID NOs: 3, 4, 5 and 6, or SEQ ID NO: 7, 8, 9 and 10.

3. The single nucleic acid of claim 1, wherein X in the type-1 single nucleic acid for detecting the single nucleotide polymorphism of codon 112 of the ApoE gene is the nucleotide sequence of SEQ ID NO: 27, or is any one nucleotide sequence selected from the group consisting of nucleotide sequences of SEQ ID NOs: 11 to 27, each comprising the first 4 to 19 nucleotides counted from the 3' end of the nucleotide sequence of SEQ ID NO: 27.

4. The single nucleic acid of claim 1, wherein X in the type-2 single nucleic acid for detecting the single nucleotide polymorphism of codon 112 of the ApoE gene is the nucleotide sequence of SEQ ID NO: 37, or is any one nucleotide sequence selected from the group consisting of nucleotide sequences SEQ ID NOs: 17 to 37, each comprising the first 10 to 29 nucleotides counted from the 3' end of the nucleotide sequence of SEQ ID NO: 37.

5. The single nucleic acid of claim 1, wherein X in the type-1 single nucleic acid for detecting the single nucleotide polymorphism of codon 158 of the ApoE gene is the nucleotide sequence of SEQ ID NO: 54, or is any one nucleotide sequence selected from the group consisting of nucleotide sequences SEQ ID NOs: 38 to 54, each comprising the first 4 to 19 nucleotides counted from the 3' end of the nucleotide sequence of SEQ ID NO: 54.

6. The single nucleic acid of claim 1, wherein X in the type-2 single nucleic acid for detecting the single nucleotide polymorphism of codon 158 of the ApoE gene is the nucleotide sequence of SEQ ID NO: 64, or is any one nucleotide sequence selected from the group consisting of nucleotide sequences SEQ ID NOs: 44 to 64, each comprising the first 10 to 29 nucleotides counted from the 3' end of the nucleotide sequence of SEQ ID NO: 64.

7. The single nucleic acid of claim 1, wherein Z in the type-1 single nucleic acid for detecting the single nucleotide polymorphism of codon 112 of the ApoE gene is the nucleotide sequence of SEQ ID NO: 84, or is any one nucleotide sequence selected from the group consisting of nucleotide sequences SEQ ID NOs: 65 to 84, each comprising the first 1 to 19 nucleotides counted from the 5' end of the nucleotide sequence of SEQ ID NO: 84.

8. The single nucleic acid of claim 1, wherein Z in the type-2 single nucleic acid for detecting the single nucleotide polymorphism of codon 112 of the ApoE gene is any one nucleotide sequence selected from the group consisting of SEQ ID NOs: 65 to 69.

9. The single nucleic acid of claim 1, wherein Z in the type-1 single nucleic acid for detecting the single nucleotide polymorphism of codon 158 of the ApoE gene is the nucleotide sequence of SEQ ID NO: 104, or is any one nucleotide sequence selected from the group consisting of nucleotide sequences SEQ ID NOs: 85 to 104, each comprising the first 1 to 19 nucleotides counted from the 5' end of the nucleotide sequence of SEQ ID NO: 104.

10. The single nucleic acid of claim 1, wherein Z in the type-2 single nucleic acid for detecting the single nucleotide polymorphism of codon 158 of the ApoE gene is any one nucleotide sequence selected from the group consisting of SEQ ID NOs: 85 to 89.

11. A kit for real-time detection of single nucleotide polymorphisms of ApoE gene, the kit comprising the single nucleic acid of any one of claims 1 to 10 and the cleavage reagent to cleave Y of the single nucleic acid when Y of the single nucleic acid hybridized with the ApoE gene,
Wherein the cleavage reagent is a ribonuclease (RNase) selected from among RNaseH, RNase II, RNase III, RNase IV and RNase T2.

12. A method for detecting single nucleotide polymorphisms of ApoE gene, the method comprising steps of:
a) isolating a target nucleic acid comprising ApoE gene single nucleotide polymorphism sites to be detected from a biological sample;
b) producing the single nucleic acid of any one of claims 1 to 10,
c) either mixing the target nucleic acid isolated in step a), the type-1 single nucleic acid produced in step b), an ApoE gene-specific primer set, and a cleavage reagent, or mixing the target nucleic acid isolated in step a), the type-2 single nucleic acid produced in step b), and a cleavage reagent, and then amplifying a target nucleic acid-single nucleic acid complex comprising the ApoE gene single nucleotide polymorphism sites by an extension reaction; and
d) measuring the amount of a single nucleic acid fragment separated from the target nucleic acid-single nucleic acid complex comprising the ApoE gene single nucleotide polymorphism sites, amplified in step c).

13. The method of claim 12, wherein the target nucleic acid comprising ApoE gene single nucleotide polymorphism sites in step a) is an RNA or DNA to be detected from the sample, or cDNA obtained by amplifying the RNA with reverse transcription polymerase.

14. The method of claim 12, wherein the cleavage reagent in step c) is a ribonuclease (RNase) selected from among RNaseH, RNase II, RNase III, RNase IV and RNase T2.

15. The method of claim 12, wherein the ApoE gene-specific primer set in step c) consists of SEQ ID NOs: 1 and 2.
